# EUROPEAN PATENT APPLICATION

(11) **EP 1 889 616 A2**
(43) Date of publication of application: **20.02.2008**
(21) Application number: 07113265.8
(22) Date of filing: 26.07.2007
(51) Int. Cl.: A61K 31/375, A61K 36/736, A61K 36/738, A61K 36/61, B01D 11/02

(54) **Extraction method of mixtures comprising ascorbic acid from a dry ground vegetable product**

(30) Priority: 26.07.2006 IT MI20061466
(71) Applicant: Farmatek S.r.l., 09132 Cagliari (IT)
(72) Inventor: Fochesato, Antonio, 36015, SCHIO (Vicenza) (IT); Paiaro, Ennio, 35142, PADOVA (IT)
(74) Representative: De Gregori, Antonella

(57) **Abstract**

An extraction method is described of mixtures comprising ascorbic acid from a dry ground vegetable product, by extraction with polar and/or semipolar solvents containing at least one dissolved gas, in the presence of a magnetic field.

## Description

The present invention relates to a method for the extraction of mixtures comprising ascorbic acid (vitamin C) from a dry ground vegetable product.

The ascorbic acid (vitamin C) which is found in nature and synthetically produced ascorbic acid (vitamin C) are completely identical as far as their chemical structure is concerned. Vitamin C or natural ascorbic acid, however, i.e. the ascorbic acid present in vegetables, is absorbed much better by living organisms: it is in fact more bioavailable.

A particular interest has therefore developed for the extraction of ascorbic acid or vitamin C from plants and in particular from dry ground vegetable products.

Dry ground vegetable products refer to vegetable preparations obtained starting from parts of the plant which are dried and then ground. Depending on the type of plant, the parts which are subjected to drying and grinding are different. In the case of Aceroles, for example, it is the peduncle of the fruit and fruit itself which are substantially dried and ground to form the dry ground vegetable product available on the market, whereas in the case of Dog Roses the flowers and leaves are dried and ground to form the dry ground vegetable product available on the market.

The methods currently used for the production of ascorbic acid or vitamin C are divided into synthesis methods of synthetic vitamin C and extraction methods of natural vitamin C. The synthesis methods of synthetic vitamin C are substantially the Reichstein method or the "two-step fermentation" method, both of which use sorbitol as starting raw material. As already mentioned, however, synthetic ascorbic acid, with a chemical structure identical to natural vitamin C, does not have the same bioavailability as natural vitamin C or ascorbic acid.

The extraction methods of natural ascorbic acid or vitamin C envisage extraction with water at 45°C, with a theoretical yield of 33% and an effective yield which does not exceed 25%.

These methods therefore do not allow an extraction of ascorbic acid from vegetable substances which is satisfactory either in qualitative and quantitative terms. Not even is the extraction method with a rotating magnetic field described in European patent application EP 1641903 applied to ascorbic acid capable of obtaining quantitatively interesting results, operating in a strongly acid environment (pH 1.5-2).

An objective of the present invention is therefore to find a new extraction method which overcomes the disadvantages of the known art described above.

This objective has been achieved with the method according to the present invention which surprisingly overcomes the drawbacks of the known art, providing a particularly effective extraction, from both a qualitative and quantitative point of view.

An object of the present invention therefore relates to an extraction method of mixtures comprising ascorbic acid from a dry ground vegetable product, by extraction with a solvent system consisting of polar and/or semipolar solvents containing at least a dissolved gas, in the presence of a magnetic field.

The magnetic field is preferably a rotating magnetic field.

In particular, the mixture extracted with the method according to the present invention contains ascorbic acid (vitamin C), bioflavonoids, preferably bioflavonoids of Acerole and Dog Rose, such as quercetin-3-alpha-O-ramnoside, kaempferol; antocyanines and antocyanidines such as malvidine-3,5-diglucoside, cyanidine-3-glucoside, pelargonidene. These mixtures also comprise phenolic acids, such as, for example, chlorogenic acid, caffeic acid, p-cumaric acid and ferulic acid.

In particular, the method according to the present invention allows "real" solutions to be prepared, starting from dry ground vegetable products rich in vitamin C (ascorbic acid), such as for example, Dog Rose, Acerole, Camu Camu.

The extraction method according to the present invention does in fact allow the preparation of aqueous solutions (90-95% H₂O), containing from 25,000 to 500,000 mg/l of ascorbic acid (vitamin C) in dissolved form, in the presence of bioflavonoids, antocyanines and terpenes.

In particular, the solutions prepared with the method according to the present invention are "real" solutions: they are in fact free of the Tyndall effect (laser light scattering). This means that all the molecules present are free to interact individually (Dalton law) according to their chemical nature.

The solvent system consisting of polar and/or semipolar solvents containing at least one dissolved gas preferably consists of water, ethanol and CO₂.

The quantity of water in the solvent system preferably varies from 70 to 95% by weight with respect to the total weight of the solvent system consisting of polar and/or semipolar solvents.

The quantity of ethanol in the solvent system preferably varies from 5 to 30% by weight with respect to the total weight of the solvent system consisting of polar and/or semipolar solvents.

The quantity of dissolved CO₂ in the solvent system preferably varies from 0.5 to 5% by weight with respect to the total weight of the solvent system consisting of polar and/or semipolar solvents.

The preferred solvent system for the extraction method according to the present invention consists of 95% by weight of distilled water, containing 1.5% by weight of dissolved CO₂ and 5% by weight of ethanol.

The use of polar solvents combined with semipolar solvents does in fact allow the extraction of a wide range of compounds having a molecular weight greater than 500 Dalton.

Even more preferably, the polar and/or semipolar solvent is ethanol, in particular pharmaceutical ethanol.

Pharmaceutical ethanol refers to extremely pure neutral ethanol 96% vol. with a maximum content of contaminants equal to 0.058 mg/l.

The magnetic field is a rotating magnetic field which has an intensity varying from 500 to 3500 Gauss, preferably from 1500 to 3000 Gauss.

The extraction is effected at a temperature ranging from 20°C to 75°C, preferably from 23°C to 60°C and is carried out for a time varying from 15 to 120 minutes, preferably from 30 to 60 minutes.

An extraction method by extraction with a preferred solvent system consisting of 95% by weight of distilled water and 5% by weight of ethanol, containing 1.5% by weight of dissolved CO₂, at a temperature varying from 23°C to 60°C, for a time ranging from 30 to 60 minutes, with a variable magnetic field ranging from 1500 to 3000 Gauss, is particularly preferred.

As observed, the method according to the present invention enables mixtures comprising ascorbic acid to be obtained, wherein all the compounds present, bioflavonoids, antocyanines and terpenes, are present as free molecules.

A fundamental advantage of the extraction method according to the present invention is that it allows solutions with different concentrations to be obtained. By varying the ratio between time/temperature/magnetic field intensity, it is in fact possible to cover the whole range of extractions of all the components of the dry ground vegetable product, soluble in the solvent system according to the present invention.

The presence of bioflavonoids, moreover, allows an increase in the absorption of ascorbic acid (vitamin C) ranging from 30 to 35% on the part of living organisms of the zoomorphous type.

All these additional compounds present in the mixture in solution, extracted with the method according to the present invention, create a synergic action with vitamin C on an antibacterial, antifungal and antiyeast level.

A further advantage of the extraction method according to the present invention is that it allows real solutions or hydrosoluble gels containing ascorbic acid and bioflavonoids to be obtained, which amazingly favour the absorption of bivalent iron in an acid environment (i.e. in the stomach). Ascorbic acid does in fact favour the absorption of the metal on a systemic level, favouring its deposit and the combination of ascorbic acid/bioflavonoids proves to have a positive synergy with respect to the antioxidizing capacity.

A further object of the present invention therefore relates to the solutions obtained with the extraction method according to the present invention, comprising ascorbic acid and free molecular structures of bioflavonoids, antocyanines and terpenes.

Solutions obtained with the extraction method according to the present invention on dry ground vegetable products of Acerole and Dog Rose, are preferred.

An object of the present invention also relates to hydrosoluble gels obtained with the extraction method according to the present invention and subsequent concentration phase in a ratio of about 4:1 under vacuum at 80-85°C, comprising ascorbic acid and free molecular structures of bioflavonoids, antocyanines and terpenes.

The real solutions or hydrosoluble gels obtained with the method according to the present invention have a high concentration of natural vitamin C or ascorbic acid, in particular up to 500,000 mg/l, consequently equal to a real solution with a concentration of 50% by weight of ascorbic acid and of particular interest from an industrial point of view.

The real solutions or hydrosoluble gels obtained with the method according to the present invention are also bacteria-free, they increase the bioavailability of the ascorbic acid by 35% and increase the iron absorption capacity on a systemic level.

The vitamin C or ascorbic acid so far used is available in the form of a finely ground powder, wherein the powders derive from the grinding of plants such as Dog Rose, Camu Camu, etc.

Like all powders, they can be dangerous both on a respiratory level and also on a flammability level: it is in fact known that micronized powders give rise to sudden fire phenomena (flash fire), due to the accumulation of electrostatic charges in a dry environment.

All powders also cause irritation of the respiratory tract upon inhalation. These powders also have a very low apparent density of about 480-500 g/l and this makes it necessary to use a high quantity of powder to obtain industrially interesting quantities of ascorbic acid.

The real solutions/hydrosoluble gels according to the present invention therefore have the advantage of not being flammable and having no health risks, at the same time being extremely practical to use.

The characteristics and advantages of the extraction method according to the present invention will be easier to understand from the following detailed illustrative description, referring to the following examples.

### Example 1

The extraction method according to the present invention was applied to a starting material consisting of Acerole in powder form with a starting content of ascorbic acid or vitamin C equal to 350,000 mg/kg (the quantity of vitamin C was determined by means of HPLC analysis, the content of bioflavonoids by means of HPLC/UV VIS, specifically according to the methods ASTM E1508 and USP 621).

The powder or dry ground product of Acerole is available on the market and, as indicated above, is obtained by the drying and grinding of peduncles of the fruit and the fruit itself of Acerole.

200 g of Acerole in powder form were put in a pyrex flask having a capacity of 1000 ml, 100 ml of ethanol were then added and the mixture was mixed for 15 minutes by means of a magnetic anchor-stirrer, coated with teflon®. 788 ml of distilled water with 12 ml of dissolved CO₂ (equal to 1.5% by weight) were then added. The flask was subsequently sealed with parafilm® and the mixture was left to rest for 30 minutes at room temperature. A rotating magnetic field was then applied for 60 minutes at 23°C, with a magnetic field intensity of 1500 Gauss and 0.29 kV of negative polarity. The mixture was then filtered with a filter of the ABET 400 type and, after filtration, mixed again for 15 minutes.

The solution obtained contains 52,000 mg/l of natural vitamin C against 70,000 mg/l contained in the starting material. The extraction yield is therefore equal to 75%.

This extraction yield has never been obtained with other methods of the known art, as the solubility of ascorbic acid or vitamin C is equal to a theoretical value of 33% in water and 63% in pure ethanol.

The solution was further concentrated in a ratio of 4:1 under vacuum at 80-85°C so as to reach a concentration equal to 208,000 mg/l. At this concentration, the product is no longer in the form of a liquid solution, but in the form of a gelatin. This gelatin or gel however is completely soluble in water at room temperature (23°C), again forming a real solution.

2,500 mg/l of bioflavonoids are present in the extraction product in the primary solution and 10,000 mg/l of bioflavonoids in the concentrated solution, bioflavonoids titrated as Galangine (3,5,7-trihydroxyflavon C₁₅H₁₂O₅).

### Example 2

The extraction method according to the present invention was applied to a starting material consisting of Dog Rose in powder form with a starting content of ascorbic acid or vitamin C equal to 210,000 mg/kg (the quantity of vitamin C was determined by means of HPLC analysis, the content of bioflavonoids by means of HPLC/UV VIS, specifically according to the methods ASTM E1508 and USP 621).

The powder or dry ground product of Dog Rose is available on the market and, as indicated above, is obtained by the drying and grinding of the flowers and leaves of Dog Rose.

200 g of Dog Rose in powder form were put in a pyrex flask having a capacity of 1000 ml, 100 ml of ethanol were then added and the mixture was mixed for 15 minutes by means of a magnetic anchor-stirrer, coated with teflon®. 788 ml of distilled water with 12 ml of dissolved CO₂ (equal to 2.05% by weight) were then added. The flask was subsequently sealed with parafilm® and the mixture was left to rest for 30 minutes at room temperature. A rotating magnetic field was then applied for 60 minutes at 23°C, with a magnetic field intensity of 1500 Gauss and 0.29 kV of negative polarity. The mixture was then filtered with a filter of the ABET 400 type and, after filtration, mixed again for 15 minutes.

The solution obtained contains 34,000 mg/l of natural vitamin C against 54,000 mg/l contained in the starting material. The extraction yield is therefore equal to 64%.

This extraction yield has never been obtained with other methods of the known art, as the solubility of ascorbic acid or vitamin C is equal to a theoretical value of 33% in water and 63% in pure ethanol.

The solution was further concentrated in a ratio of 4:1 under vacuum at 80-85°C so as to reach a concentration equal to 136,000 mg/l. At this concentration, the product is no longer in the form of a liquid solution, but in the form of a gelatin. This gelatin or gel however is completely soluble in water at room temperature (23°C), again forming a real solution.

1,500 mg/l of bioflavonoids are present in the extraction product in the primary solution and 6,000 mg/l of bioflavonoids in the concentrated solution, bioflavonoids titrated as Galangine (3,5,7-trihydroxyflavon C₁₅H₁₂O₅).

### Example 3

The extraction method according to the present invention was applied to a starting material consisting of Acerole in powder form with a starting content of ascorbic acid or vitamin C equal to 350,000 mg/kg (the quantity of vitamin C was determined by means of HPLC analysis, the content of bioflavonoids by means of HPLC/UV VIS, specifically according to the methods ASTM E1508 and USP 621).

400 g of Acerole in powder form or dry ground Acerole were put in a pyrex flask having a capacity of 1,000 ml, 100 ml of ethanol were then added and the mixture was mixed for 15 minutes by means of a magnetic anchor-stirrer, coated with teflon®. 588 ml of distilled water with 12 ml of dissolved CO₂ (equal to 1.05% by weight) were then added. The flask was subsequently sealed with parafilm® and the mixture was left to rest for 30 minutes at room temperature. A rotating magnetic field was then applied for 60 minutes at 23°C, with a magnetic field intensity of 1,500 Gauss and 0.29 kV of negative polarity. The mixture was then filtered with a filter of the ABET 400 type and, after filtration, mixed again for 15 minutes.

The solution obtained contains 110,000 mg/l of natural vitamin C against 140,000 mg/l contained in the starting material. The extraction yield is therefore equal to 77%.

This extraction yield has never been obtained with other methods of the known art, as the solubility of ascorbic acid or vitamin C is equal to a value of 33% (theoretical) in water and 63% in pure ethanol.

The product is in liquid form as a real solution.

5,000 mg/l of bioflavonoids, titrated as Galangine (3,5,7-trihydroxyflavon C₁₅H₁₂O₅), are present in the extraction product.

### Example 4

The extraction method according to the present invention was applied to a starting material consisting of Dog Rose in powder form with a starting content of ascorbic acid or vitamin C equal to 270,000 mg/kg (the quantity of vitamin C was determined by means of HPLC analysis, the content of bioflavonoids by means of HPLC/UV VIS, specifically according to the methods ASTM E1508 and USP 621).

400 g of Dog Rose in powder form or dry ground Dog Rose were put in a pyrex flask having a capacity of 1,000 ml, 100 ml of ethanol were then added and the mixture was mixed for 15 minutes by means of a magnetic anchor-stirrer, coated with teflon®. 588 ml of distilled water with 12 ml of dissolved CO₂ (equal to 1.05% by weight) were then added. The flask was subsequently sealed with parafilm® and the mixture was left to rest for 30 minutes at room temperature. A rotating magnetic field was then applied for 60 minutes at 23°C, with a magnetic field intensity of 1,500 Gauss and 0.29 kV of negative polarity. The mixture was then filtered with a filter of the ABET 400 type and, after filtration, mixed again for 15 minutes.

The solution obtained contains 75,000 mg/l of natural vitamin C against 108,000 mg/l contained in the starting material. The extraction yield is therefore equal to 70%.

This extraction yield has never been obtained with other methods of the known art, as the solubility of ascorbic acid or vitamin C is equal to a value of 33% (theoretical) in water and 63% in pure ethanol.

3,000 mg/l of bioflavonoids, titrated as Galangine (3, 5, 7-trihydroxyflavon C₁₅H₁₂O₅), are present in the extraction product.

The increase in the quantity of CO₂ dissolved in water and the consequent relative increase in the quantity of carbonic acid, improve the solvent capacity of the water and ethanol with the same magnetic field intensity. This effect is absolutely surprising and can be linked to the increase in polarity of the ternary solvent mixture.

The solvent capacity of a mixture consisting of 90% by weight of water and 10% by weight of ethanol normally does not exceed vitamin C extraction with an extraction yield of 36%, whereas the extraction capacity of the same solvent system with the method according to the present invention allows an extraction yield of 77% to be obtained.

### Example 5

### BACTERIA

The starting materials consisting of dry ground product of Acerole and Dog Rose had the following bacterial charge:

| | |
|---|---|
| ACEROLE IN POWDER FORM | 1,000 cfu/g; |
| DOG ROSE IN POWDER FORM | 1,000 cfu/g. |

After application of the extraction method according to the present invention described in Examples 1-4, the liquid products and gels showed the following contents of bacterial charge:
ACEROLE, real solution, concentration 52,000 mg/l: 0 cfu/g;
ACEROLE, gel, concentration 208,000 mg/l: 0 cfu/g;
DOG ROSE, real solution, concentration 34,000 mg/l: 0 cfu/g;
DOG ROSE, gel, concentration 136,000 mg/l: 0 cfu/g;
ACEROLE, concentrated solution, 104,000 mg/l: 0 cfu/g;

It is therefore evident that the extraction method according to the present invention allows the bacterial charges present in the raw material to be lowered and/or annulled.

### YEASTS AND MILDEW

The starting materials consisting of dry ground products of Acerole and Dog Rose had the following charge of yeasts and mildew:

| | |
|---|---|
| ACEROLE IN POWDER FORM | 50 cfu/g; |
| DOG ROSE IN POWDER FORM | 50 cfu/g. |

After application of the extraction method according to the present invention described in Examples 1-4, the liquid products and gels showed the following contents of yeasts and mildew:
ACEROLE, real solution, concentration 52,000 mg/l: 0 cfu/g;
ACEROLE, gel, concentration 208,000 mg/l: 0 cfu/g;
DOG ROSE, real solution, concentration 34,000 mg/l: 0 cfu/g;
DOG ROSE, gel, concentration 136,000 mg/l: 0 cfu/g;

It is therefore evident that the extraction method according to the present invention allows the bacterial charges, mildew and yeasts present in the raw material to be lowered and/or annulled, obtaining solutions or gels free of bacterial charge, mildew or yeasts.

### Example 6 (comparative)

1 kg of dry ground product of Acerole with a density of 480 g/l, has a content of ascorbic acid of 350,000 mg/kg. The volume expressed in ml indicates that 1 kg is equal to 2200 ml i.e. 2.2 1.

In order to obtain a concentration of ascorbic acid equal to 290,000 mg/l, it is therefore necessary to have a powder volume of 1,800 ml, therefore 1.8 1 against a volume of 1 1 of vitamin C in solution according to the present invention.

In order to produce a tablet containing 180 mg it is necessary to use 11.31 ml of vitamin C in powder form against 6.10 ml of real solution at 50% by weight.

This aspect is extremely advantageous for all utilizations which envisage the use of natural and non-synthetic ascorbic acid.

## Claims

1. An extraction method of mixtures comprising ascorbic acid from dry ground vegetable product, by extraction with a solvent system consisting of polar and/or semipolar solvents containing at least one dissolved gas, in the presence of a magnetic field.

2. The extraction method according to claim 1, **characterized in that** the magnetic field is a rotating magnetic field.

3. The extraction method according to claim 1, **characterized in that** the mixture extracted comprises ascorbic acid (vitamin C), bioflavonoids, antocyanines, antocyanidines, terpenes and phenolic acids.

4. The extraction method according to claim 1, **characterized in that** the mixture extracted is in the form of a "real" solution in which all the molecules present are free to interact individually (Dalton law) according to their chemical nature.

5. The extraction method according to claim 1, **characterized in that** the dry ground vegetable product rich in vitamin C (ascorbic acid) is selected from dry ground vegetable products of Dog Rose, Acerole and Camu Camu.

6. The extraction method according to claim 1, **characterized in that** the mixture extracted is in the form of an aqueous solution (90-95% H₂O), containing from 25,000 to 500,000 mg/l of ascorbic acid (vitamin C) in dissolved form, in the presence of bioflavonoids, antocyanines and terpenes.

7. The extraction method according to claim 1, **characterized in that** the solvent system consists of water, ethanol and CO₂.

8. The extraction method according to claim 7, **characterized in that** the quantity of water in the solvent system varies from 70 to 95% by weight with respect to the total weight of the solvent system consisting of polar and/or semipolar solvents.

9. The extraction method according to claim 7, **characterized in that** the quantity of ethanol in the solvent system varies from 5 to 30% by weight with respect to the total weight of the solvent system consisting of polar and/or semipolar solvents.

10. The extraction method according to claim 7, **characterized in that** the quantity of CO₂ dissolved in the solvent system varies from 0.5 to 5% by weight with respect to the total weight of the solvent system consisting of polar and/or semipolar solvents.

11. The extraction method according to claim 1, **characterized in that** the solvent system consists of 95% by weight of distilled water containing 1.5% by weight of dissolved CO₂ and 5% by weight of ethanol.

12. The extraction method according to claim 1, **characterized in that** the rotating magnetic field has an intensity varying from 500 to 3,500 Gauss, preferably from 1,500 to 3,000 Gauss.

13. The extraction method according to claim 1, **characterized in that** the extraction is effected at a temperature ranging from 20°C to 75°C, preferably from 23°C to 60°C and is carried out for a time varying from 15 to 120 minutes, preferably from 30 to 60 minutes.

14. The extraction method according to claim 1, **characterized in that** the extraction is effected by means of a solvent system consisting of 95% by weight of distilled water and 5% by weight of ethanol, containing 1.5% by weight of dissolved CO₂, at a temperature varying from 23°C to 60°C, for a time ranging from 30 to 60 minutes, with a variable magnetic field ranging from 1,500 to 3,000 Gauss.

15. Solutions obtainable with the method according to any of the previous claims, **characterized in that** they contain ascorbic acid and free molecular structures of bioflavonoids, antocyanines, antocyanidines and terpenes.

16. The solutions according to claim 15, **characterized in that** they are obtained starting from dry ground products of Acerole and Dog Rose.

17. The solutions according to claim 15 or 16, **characterized in that** they contain a concentration of natural vitamin C or ascorbic acid of up to 500,000 mg/l, therefore equal to a solution with a concentration of 50% by weight of ascorbic acid.

18. The solutions according to any of the claims from 15 to 17, **characterized in that** they are free of bacteria.

19. The solutions according to any of the claims from 15 to 17, **characterized in that** they increase the bioavailability of ascorbic acid by 35%.

20. The solutions according to any of the claims from 15 to 17, **characterized in that** they increase the absorption capacity of iron on a systemic level.

21. Hydrosoluble gels obtainable with the method according to any of the previous claims from 1 to 14 and subsequent concentration phase in a ratio of about 4:1 under vacuum at 80-85°C, **characterized in that** they contain ascorbic acid, bioflavonoids, antocyanines and terpenes.

22. The gels according to claim 21, **characterized in that** they are obtained starting from dry ground products of Acerole and Dog Rose.

23. The gels according to claim 21 or 22, **characterized in that** they contain a concentration of natural vitamin C or ascorbic acid of up to 500,000 mg/l, therefore equal to a solution with a concentration of 50% by weight of ascorbic acid.

24. The gels according to any of the claims from 21 to 23, **characterized in that** they are free of bacteria.

25. The gels according to any of the claims from 21 to 23, **characterized in that** they increase the bioavailability of ascorbic acid by 35%.

26. The gels according to any of the claims from 21 to 23, **characterized in that** they increase the absorption capacity of iron on a systemic level.
